(19) Europäisches Patentamt
European Patent Office
Office européen des brevets

(11) **EP 4 194 843 B1**

(12) **EUROPEAN PATENT SPECIFICATION**

(45) Date of publication and mention
of the grant of the patent:
**25.03.2026 Bulletin 2026/13**

(21) Application number: **22210408.5**

(22) Date of filing: **30.11.2022**

(51) International Patent Classification (IPC):
***G01N 23/201*** (2018.01)

(52) Cooperative Patent Classification (CPC):
**G01N 23/201;** G01N 2223/612

(54) **THREE-DIMENSIONAL ELECTRON DENSITY MAP SPECIFYING APPARATUS, SYSTEM, METHOD, AND PROGRAM**

VORRICHTUNG, SYSTEM, VERFAHREN UND PROGRAMM ZUR ANGABE EINER DREIDIMENSIONALEN ELEKTRONENDICHTEKARTE

APPAREIL, SYSTÈME, PROCÉDÉ ET PROGRAMME DE SPÉCIFICATION DE CARTE DE DENSITÉ D'ÉLECTRONS TRIDIMENSIONNELLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **10.12.2021 JP 2021200965**
**31.10.2022 JP 2022174381**

(43) Date of publication of application:
**14.06.2023 Bulletin 2023/24**

(73) Proprietor: **Rigaku Corporation**
**Akishima-shi**
**Tokyo 196-8666 (JP)**

(72) Inventors:
• **SATO, Takashi**
**Tokyo, 196-8666 (JP)**
• **MATSUMOTO, Takashi**
**Tokyo, 196-8666 (JP)**
• **HASEGAWA, Tomokazu**
**Tokyo, 196-8666 (JP)**

(74) Representative: **Lambacher, Michael et al**
**V. Füner Ebbinghaus Finck Hano**
**Patentanwälte**
**Mariahilfplatz 3**
**81541 München (DE)**

(56) References cited:
• **GRANT THOMAS D: "Ab initio electron density determination directly from solution scattering data", NATURE METHODS, vol. 15, no. 3, 29 January 2018 (2018-01-29), pages 191 - 193, XP037398763, ISSN: 1548-7091, DOI: 10.1038/ NMETH.4581**
• **THOMAS D GRANT: "Video: Convergence process of electron density reconstruction from SAXS data", 29 January 2018 (2018-01-29), XP093035139, Retrieved from the Internet <URL:https://www.nature.com/articles/ nmeth.4581> [retrieved on 20230328]**
• **RONNEBAUM TREY A ET AL: "Higher-order oligomerization of a chimeric [alpha][beta] [gamma] bifunctional diterpene synthase with prenyltransferase and class II cyclase activities is concentration-dependent", JOURNAL OF STRUCTURAL BIOLOGY, ACADEMIC PRESS, UNITED STATES, vol. 210, no. 1, 21 January 2020 (2020-01-21), XP086081580, ISSN: 1047-8477, [retrieved on 20200121], DOI: 10.1016/ J.JSB.2020.107463**
• **FAN HAIFU ET AL: "Applications of direct methods in protein crystallography for dealing with diffraction data down to 5?◆ resolution", ACTA CRYSTALLOGRAPHICA SECTION A FOUNDATIONS AND ADVANCES, vol. 70, no. 3, 12 March 2014 (2014-03-12), pages 239 - 247, XP093035135, DOI: 10.1107/S2053273313034864**

**Description**

RELATED ART

Field of the Invention

The present invention relates to a three-dimensional electron density map specifying apparatus, system, method, and program for specifying a three-dimensional electron density map of a macromolecule in a solution.

Description of the Related Art

Observation methods of a biomacromolecule in a solution have been studied further. When X-rays are applied to biomacromolecules that are in a state free to move in a solution, a ring-shaped scattered ray is generated instead of a spot. A technique is known in which such a scattered ray is detected, and a three-dimensional electron density map of a target molecule is obtained from the acquired measured X-ray scattering profile (Non-Patent Document 1).
In the method described in Non-Patent Document 1, the volume of a cube in a real space containing particles is represented by a voxel of a cube discretized in an $N \times N \times N$ grid, and an electron density map is calculated by iteratively searching for a structural factor based on X-ray scattering data acquired from a sample.

Non-patent Documents

Non-patent Document 1: Thomas D Grant, "Ab initio electron density determination directly from solution scattering data", Nature Methods volume 15, 29 January 2018, pages 191-193
However, in the method described in Non-Patent Document 1, only an electron density map resulting from averaging a plurality of electron density maps is obtained. Therefore, when analyzing the form of a rigid molecule, a reasonable electron density map is calculated. However, in the case of a flexible molecule, the form of dynamic structures is averaged, and the electron density to be originally obtained lacks the characteristics of the molecule and has little meaning.

Non-patent document 2: Ronnebaum Trey A. et al.,« Higher-order oligomerization of a chimeric $\alpha\beta\gamma$ bifunctional deterpene synthase with prenyltransferase and class II cyclase activities is concentration-dependent", Journal Of Structural Biology, vol. 210, 21 January 2020, Article number: 107463, pages 1-11 describes, in particular, using small-angle X-ray scattering to provide complementary information regarding size and shape of copalyl diphosphate synthase from the fungus *Penicillium verruculosum* (PvCPS).

SUMMARY OF THE INVENTION

The present invention has been made in view of such circumstances, and an object of the present invention is to provide an electron density map specifying apparatus, system, method, and a program capable of reproducing an electron density map of a macromolecule in a solution having a flexible nature and showing structure with dynamic fluctuation.

(1) In order to achieve the above object, the electron density map specifying apparatus of the present invention is an electron density map specifying apparatus for specifying an electron density map of a macromolecule in a solution, said electron density map being represented by a plurality of discretised voxels, said voxels having a particular voxel size, and said apparatus comprises an electron density map generating section for (i) calculating a theoretical X-ray scattering profile calculated by randomly assigning an electron density to each voxel in the real space, (ii) calculating scaling structure factors to fit the calculated theoretical X-ray scattering profile with an X-ray scattering profile obtained by measuring a sample, (iii) providing a new electron density in real space using the scaled structure factors, (iv) repeating the calculation of the structure factors and the electron density until convergence to generate one electron density map, and (v) generating the one electron density map a predetermined number of times for said particular voxel size to generate multiple electron density maps, an index calculating section for calculating $\chi^2$ defined by formula (1) below as an index representing a degree of coincidence between the respective calculated theoretical X-ray scattering profiles and the measured X-ray scattering profile, $\chi^2$ being calculated said predetermined number of times, and an electron density map selecting section for selecting an electron density map having a correlation between a first plot of a parameter representing a molecular size with respect to the calculated index and a regression line of the first plot and having the calculated index close to 1 as a representative electron density map from the plurality of electron density maps based on the predetermined number of calculated indexes, wherein

$$\chi 2 = \left[ \frac{I_{\text{exp}}^{(i)}(q_j) - I_{\text{calc}}^{(i)}(q_j)}{\sigma(q_j)} \right]^2 \quad \cdots (1)$$

where Icalc(q) is the calculated scattering intensity, Iexp(q) is the actual measurement value and $\sigma$(q) is the standard deviation of Iexp(q).

(2) Further, the electron density map specifying apparatus of the present invention further comprises a correlation determining section for generating a first plot of a parameter representing a molecular size with respect to the calculated index for each of the plurality of electron density maps by using a correlation coefficient and determining the presence or absence of correlation between the first plot and a regression line thereof, wherein the electron density map selecting section does not select the representative electron density map when there is no meaningful correlation of the first plot.

(3) Further, the electron density map specifying apparatus of the present invention further comprises a trend analyzing section for performing multivariate analysis on the distribution of the first plot when there is no correlation between the first plot and the regression line thereof, wherein the trend analyzing section determines whether there is any trend in the first plot through the multivariate analysis, and the electron density map generating section generates the electron density map again by changing the condition if it is determined that there is a trend.

(4) Further, in the electron density map specifying apparatus of the present invention, the correlation determining section generates an outputable first plot when there is no correlation between the first plot and the regression line thereof, and the electron density map generating section generates the plurality of electron density maps under a condition based on an instruction from a user.

(5) Further, in the electron density map specifying apparatus of the present invention, the electron density map generating section generates each of the plurality of electron density maps one by one in a repetitive process according to a setting.

(6) Further, in the electron density map specifying apparatus of the present invention, the electron density map generating section generates each of the plurality of electron density maps at a time in parallel processes according to a setting.

(7) Further, the electron density map specifying apparatus of the present invention further comprises a theoretical size calculating section for generating a second plot of a parameter representing a molecular size calculated based on the plurality of electron density maps with respect to each voxel size and calculating a calculated value of a parameter representing a molecular size of a macromolecule in the solution using the second plot, when each of the electron density maps is represented by a plurality of discretized voxels, and an actual size calculating section for calculating a parameter representing a molecular size of a macromolecule in the solution from the measured X-ray scattering profile as an actual measurement value, wherein the electron density map selecting section does not select the representative electron density map unless a difference between the calculated value and the actual measurement value is within a predetermined range.

(8) Further, the system of the present invention comprises an X-ray solution scattering apparatus and the electron density map specifying apparatus according to any one of (1) to (7), wherein the electron density map specifying apparatus specifies an electron density map of a macromolecule in the solution based on an X-ray scattering profile of the macromolecule in the solution measured by the X-ray solution scattering apparatus.

(9) Further, the method of the present invention is an electron density map specifying computer-implemented method for specifying an electron density map of a macromolecule in a solution, said electron density map being represented by a plurality of discretized voxels, said voxels having a particular voxel size, and comprises the steps of (i) calculating a theoretical X-ray scattering profile calculated by randomly assigning an electron density to each voxel in the real space, (ii) calculating scaling structure factors to fit the calculated theoretical X-ray scattering profile with an X-ray scattering profile obtained by measuring a sample, (iii) providing a new electron density in real space using the scaled structure factors, (iv) repeating the calculation of the structure factors and the electron density until convergence to generate one electron density map, (v) generating the one electron density map a predetermined number of times for said particular voxel size to generate multiple electron density maps, calculating $\chi^2$ defined by formula (1) below as an index representing a degree of coincidence between the respective calculated theoretical X-ray scattering profiles and the measured X-ray scattering profile, $\chi^2$ being calculated said predetermined number of times, and selecting an electron density map having a correlation between a first plot of a parameter representing a molecular size with respect to the calculated index and a regression line of the first plot and having the calculated index close to 1 as a representative electron density map from the plurality of electron density maps based on the predetermined number of calculated indexes, wherein

$$\chi 2 = \left[ \frac{I_{exp}^{(i)}(q_j) - I_{calc}^{(i)}(q_j)}{\sigma(q_j)} \right]^2 \quad \cdots (1)$$

where Icalc(q) is the calculated scattering intensity, Iexp(q) is the actual measurement value and $\sigma$(q) is the standard deviation of Iexp(q).

(10) Further, the program of the present invention is an electron density map specifying computer program for specifying an electron density map of a macromolecule in a solution and causes a computer to carry out the method of (9).

Brief Description of the Drawings

Fig. 1 is a schematic diagram showing the system of the present invention.
Fig. 2 is a perspective view showing an X-ray solution scattering apparatus.
Fig. 3 is a block diagram showing the system of the present invention.
Fig. 4 is a flowchart showing the operation of the electron density map specifying apparatus of the present invention.
Fig. 5 is a graph showing an example of an X-ray scattering profile.
Fig. 6 is a schematic diagram showing the electron density map.
Figs. 7A and 7B are graphs showing ideal and unanalyzable distributions of $\chi^2$-Rg(ind) respectively.
Fig. 8 is a graph showing a distribution of Rg(ind) with respect to $\chi^2$ of the examples.
Fig.9 is a graph showing extrapolation of Rg(sect) with respect to voxel size in the embodiment.
Fig. 10 is a list showing electron density maps and processing results in the example.
Figs. 11A to 11C are a front, plan and right side view showing the selected electron density map respectively. In order to help understand the obtained electron density map, a structural ribbon model obtained separately by X-ray crystal structure analysis is superposed.

DETAILED DESCRIPTION OF THE INVENTION

Next, embodiments of the present invention are described with reference to the drawings. To facilitate understanding of the description, the same reference numerals are assigned to the same components in the respective drawings, and duplicate descriptions are omitted.

[Electron Density Map Specific System]

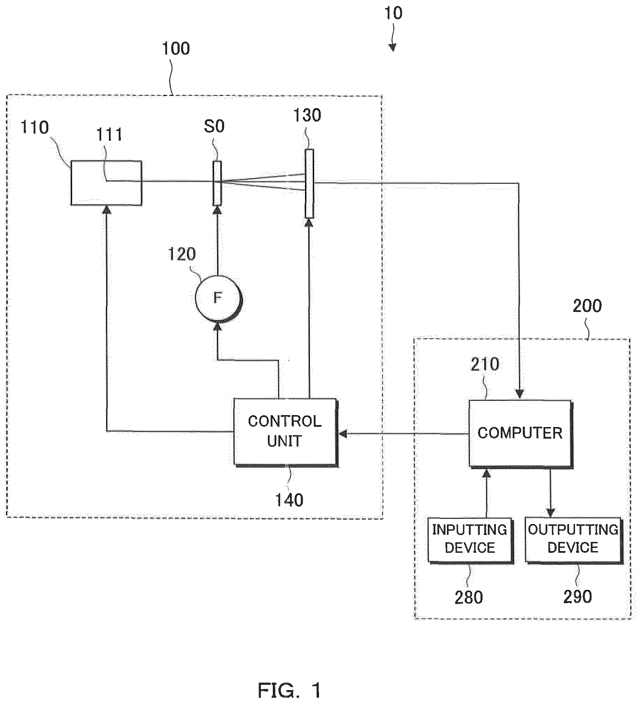

Fig. 1 is a schematic diagram showing the electron density map specifying system 10. The electron density map specifying system 10 comprises an X-ray solution scattering apparatus 100 and an electron density map specifying apparatus 200. The X-ray solution scattering apparatus 100 measures the X-ray solution scattering profile by irradiating the sample S0 with X-rays and detecting the scattered X-rays. The sample S0 is suitable to be macromolecules in solutions, in particular biomacromolecules. The sample S0 may be a pharmaceutical molecule, molecular complex or structure in solution. Further, by using the X-ray solution scattering method, it is possible to visualize an electron density corresponding to an ensemble of the structure of the biomacromolecule which is not observable in the freezing or crystalline state.

The electron density map specifying apparatus 200 comprises a computer 210, an inputting device 280, and an outputting device 290, and controls the operation of the X-ray solution scattering apparatus 100 and acquires measurement data from the X-ray solution scattering apparatus 100 and processes the data.

The X-ray solution scattering apparatus 100 comprises an X-ray generation unit 110, a sample loading mechanism 120, a detector 130, and a control unit 140. The X-ray generation unit 110 has an X-ray source 111 and irradiates the sample S0 with X-rays. The sample loading mechanism 120 delivers a sample solution to an X-ray irradiation position or a sample solution eliminating the macromolecule component. The detector 130 detects X-rays scattered by the sample S0 and transmits the acquired measured data to the computer 210.

The computer 210 is, for example, a PC, and comprises a processor that executes processes, a memory that stores programs and data, a hard disk, and the like. The computer 210 receives user input from an inputting device 280 such as a keyboard or a mouse. Meanwhile, the computer 210 displays a plot, a visualized electron density map, an input screen, and the like on an outputting device 290 such as a display. The computer 210 may be a server device placed on a cloud. In addition, from the viewpoint of processing load, the function for controlling the operation of the X-ray solution scattering apparatus 100 and the function for processing the measured data may be separated, and the control may be executed by a PC placed at the site, and the data processing may be executed by the server device.

[X-ray Solution Scattering Apparatus]

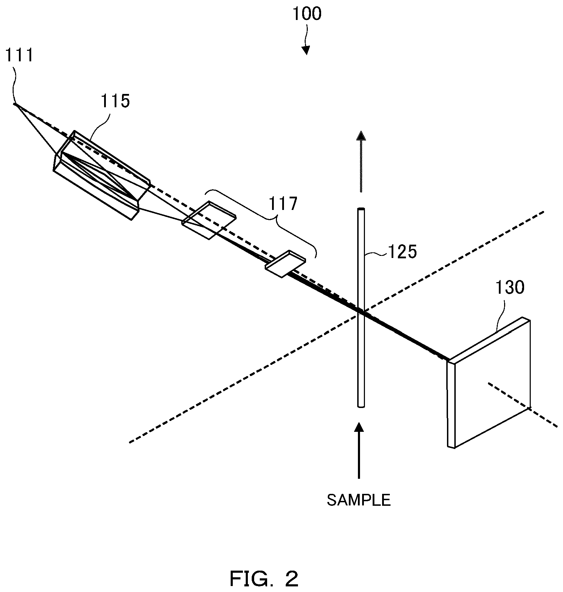

Fig. 2 is a perspective view showing the X-ray solution scattering apparatus 100. The X-ray solution scattering apparatus 100 comprises an X-ray source 111, an optical system 115, a Kratky block 117, a sample holding tube 125, and a detector 130. The X-ray source 111 is a line radiation source or a point radiation source and emits a divergent beam. The optical system 115 is, for example, a KB parallel-type or series-type optical system. A pair of Kratky blocks 117 interact with the X-rays by their respective edges to define one side and the other side of the X-ray beam. Thus, parasitic scattering can be removed from the irradiated X-rays. The sample holding tube 125 delivers and holds $1\mu l$ to $20\mu l$ of the solution sample. The detector 130 detects X-rays scattered by the solution sample.

[Electron Density Map Specifying Apparatus]

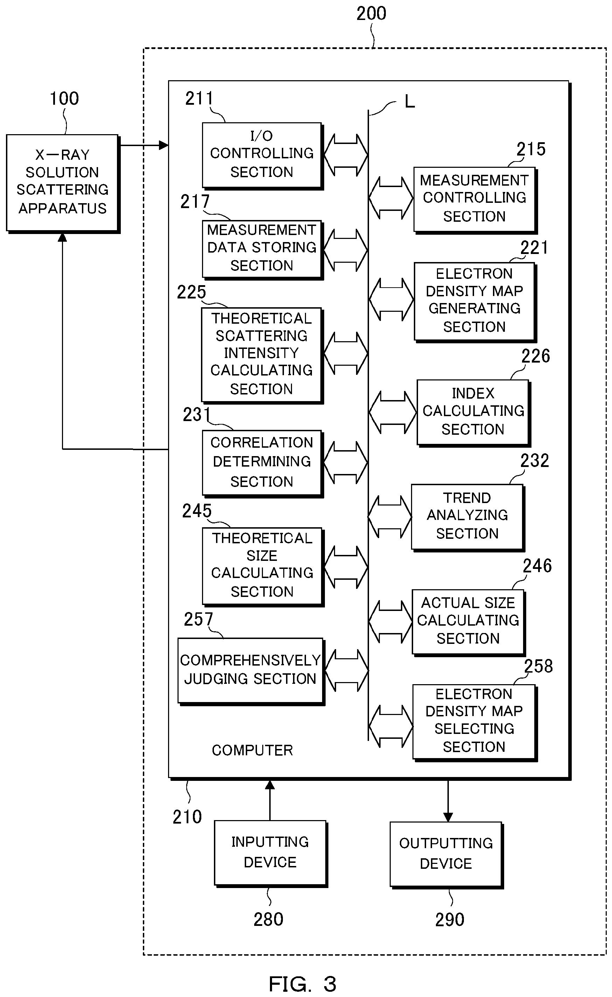

Fig. 3 is a block diagram showing the electron density map specifying system 10. The electron density map specifying apparatus 200 acquires data of the X-ray scattering profile measured by the X-ray solution scattering apparatus 100 and specifies an electron density map of the macromolecule based on the data. The functions of the electron density map specifying apparatus 200 are mainly realized by a computer 210.

The computer 210 comprises an I/O controlling section 211, a measurement controlling section 215, a measurement data storing section 217, an electron density map generating section 221, a theoretical scattering intensity calculating section 225, an index calculating section 226, a correlation determining section 231, a trend analyzing section 232, a theoretical size calculating section 245, an actual size calculating section 246, a comprehensively judging section 257, and an electron density map selecting section 258. Each section can transmit and receive information via the control bus L.

The I/O controlling section 211 receives an input from the inputting device 280 and controls an output to the outputting device 290. The I/O controlling section 211 can receive, for example, an input of a measurement condition or an input of a condition for generating a plurality of electron density maps. Further, the I/O controlling section 211 can output various plots or output an electron density map of the specified macromolecule.

The measurement controlling section 215 controls the operations of the X-ray solution scattering apparatus 100. The controls are for sample delivery, X-ray generation, and sample position and detector movement. The control instruction is transmitted to the control unit 140 in the X-ray solution scattering apparatus 100, and thus each section of the X-ray solution scattering apparatus 100 is controlled.

The measurement data storing section 217 stores measurement data of the X-ray solution scattering profile detected by the X-ray solution scattering apparatus 100. The stored measurement data is used for generating of an electron density map, calculation of an index, and calculation of an actual measurement value of a molecular size of a macromolecule.

The electron density map generating section 221 generates a plurality of electron density maps from the measured X-ray scattering profile acquired by measuring the sample S0. Details of generating of the electron density map are described later.

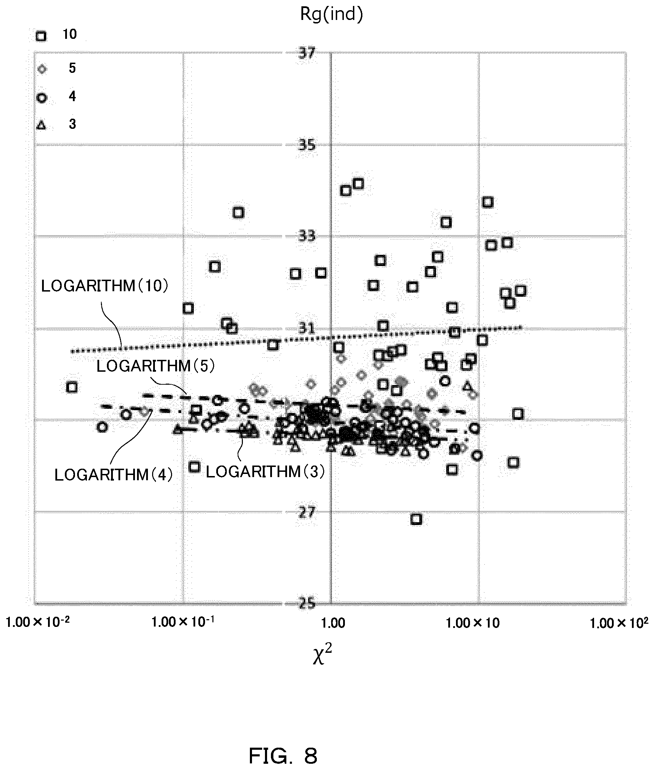

The first plot (see Fig. 8 illustrated later) can be obtained by plotting Rg(ind) values, which indicate the calculated hydrodynamic radius of gyration from an electron density map, to the values of $\chi^2$ indicating the degree of coincidence among the measured scattering curves and those calculated from the electron density maps. The hydrodynamic radius of gyration Rg of the molecule is a preferable example of a parameter representing the molecular size and may be another parameter representing the molecular size.

The electron density map generating section 221 preferably generates a plurality of electron density maps after changing the condition when the distribution of the first plot has a trend. Thus, it is possible to try to regenerate the electron density map when none of the electron density maps is valid. The electron density map generating section 221 may generate a plurality of electron density maps under a condition based on an instruction from the user. Thus, the electron density map can be tried to be generated by changing the condition when none of the electron density maps are valid.

The electron density map generating section 221 can generate each of the plurality of electron density maps one by one in a repetitive process according to the setting. Thus, it is possible to proceed with the processing by allocating necessary computational resources while reducing unnecessary processing and confirming the validity of the electron density map. Further, the electron density map generating section 221 may generate each of the plurality of electron density maps at one time in parallel processing according to the setting. Thus, the electron density map of the biomacromolecule with high validity can be reproduced in a short time.

The index calculating section 226 calculates an index indicating the degree of coincidence between the X-ray scattering profile calculated from each of the plurality of electron density maps and the measured X-ray scattering profile. Specifically, $\chi^2$ is preferable as an index for which the statistical process has been performed, but the index is not particularly limited as long as it is an index indicating the degree of coincidence with the X-ray scattering profile. In addition to $\chi^2$, a parameter of a normal distribution or a Poisson distribution, R values, RMS values, and RMD values of an index indicating the degree of coincidence between the structural factors obtained from the measured diffraction data and the structural factors based on the electron density obtained from the analysis, and the like are included. According to the present invention, $\chi^2$ is calculated as follows.

$$\chi 2 = \left[ \frac{I_{\text{exp}}^{(i)}(q_j) - I_{\text{calc}}^{(i)}(q_j)}{\sigma(q_j)} \right]^2 \quad \cdots (1)$$

where $I_{\text{calc}}$ (q) is the calculated scattering intensity, $I_{\text{exp}}$ (q) is the actual measurement value and $\sigma(q)$ is the standard deviation of $I_{\text{exp}}(q)$.

The correlation determining section 231 generates a first plot by plotting a parameter representing the molecular size with respect to the calculated index for each of the plurality of electron density maps and determines the presence or absence of correlation of the first plot. The correlation determining section 231 preferably generates an outputable first plot and shows the first plot on a display. Thus, the user can visually confirm the presence or absence of correlation in the first plot.

In the case where the repetitive processing is performed, the correlation determining section 231 preferably determines whether or not there is a correlation in the plot for each round of the repetitive processing. Thus, the processing can be proceeded while confirming the validity of the electron density map for each round of the repetitive processing. As a result, the processing can be efficiently performed when the computational resources are limited.

The trend analyzing section 232 performs multivariate analysis on the distribution of the first plot when there is no correlation in the first plot. Examples of the multivariate analysis include MCA(Multiple correspondence analysis) and PCA (principal component analysis). Thus, for example, it is possible to determine the presence or absence of any trend even if there is no correlation. For example, even when there is no correlation in the whole data, it is possible to use various types of data as correlated data by separating a plurality of types of correlated data.

The theoretical size calculating section 245 generates a second plot by plotting a parameter representing the molecular size calculated based on the plurality of electron density maps with respect to voxel sizes. The parameter representing the calculated molecular size is preferably Rg(sect) as an intercept at $\chi^2=1$ on the regression line of Rg(ind) as a calculated radius of gyration. Then, the theoretical size calculating section 245 calculates Rg(calc) as a calculated value of a parameter representing the molecular size of the macromolecule in the solution regardless of the voxel size using the second plot.

The actual size calculating section 246 calculates a parameter representing the molecular size of the macromolecule in the solution from the measured X-ray scattering profile as an actual measurement value. Specifically, a Guinier plot can be performed to calculate the Rg(exp) as the actual measurement value of the hydrodynamic radius of gyration of the molecule. If there is a sample with hydration shells in the solution, it is preferable to treat a value obtained by subtracting a predetermined value for the thickness of the hydration sphere from a value obtained from the Guinier plot as an actual measurement value Rg(exp).

The comprehensively judging section 257 determines whether or not there is a statistically significant difference between the calculated value Rg(calc) of the radius of gyration calculated by the theoretical size calculating section 245 and the actual measurement value Rg(exp) calculated by the actual size calculating section 246. When it is determined that there is no significant difference, a representative electron density map is selected from a plurality of electron density maps. If it is determined that there is a significant difference, the process is terminated.

The electron density map selecting section 258 selects a representative electron density map from the plurality of electron density maps based on the calculated index. According to the present invention, an electron density map being in accordance with Rg-$\chi^2$ correlations of the plurality of electron density maps obtained and having a $\chi^2$ close to 1 is selected as a representative electron density map. Since the representative electron density map is selected based on the index, the representative electron density map of the macromolecule in the solution which has a flexible nature and showing structure with dynamic fluctuation can be reproduced accurately. As a result, biomacromolecules in a solution without any prior information can be accurately visualized.

It is preferable that the electron density map selecting section 258 does not select a representative electron density map when there is no correlation in the first plot. Thus, if none of the electron density maps is valid, specification of the electron density map can be stopped, and unnecessary calculation can be omitted. Then, according to the situation, the electron density map can be generated again, or the other experiments are conducted again for the electron density map(s).

The electron density map selecting section 258 preferably does not select a representative electron density map unless the difference between the calculated value and the actual measurement value is within a predetermined range. Thus, even when the electron density map can be specified, the specification of the electron density map can be stopped when the electron density map is not guaranteed to be valid in terms of the molecular size.

[Electron Density Map Specifying Method]

(Entire Method)

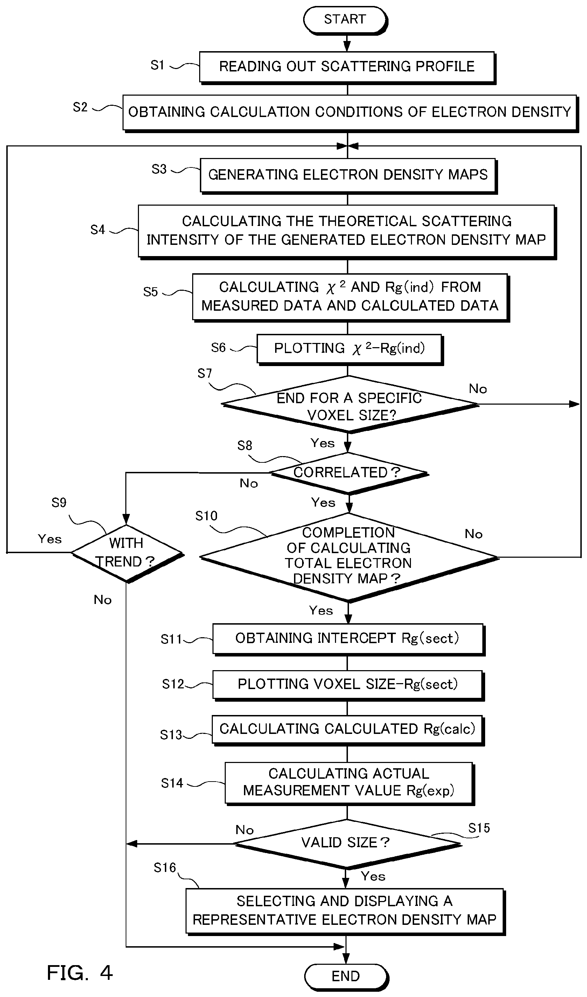

A method of specifying an electron density map of a macromolecule in a solution using the electron density map specifying system 10 configured as described above is described here. Fig. 4 is a flowchart showing the operation of the electron density map specifying apparatus 200. First, the X-ray solution scattering apparatus 100 delivers the solution with the sample S0 to a predetermined position and irradiates the sample S0 with X-rays. The X-ray solution scattering apparatus 100 detects the scattered X-rays and transmits the detected data to the computer 210 as data of an X-ray scattering profile. The computer 210 stores data of the received X-ray scattering profile.

The computer 210 reads out the X-ray scattering profile acquired according to the sample S0 for which the electron density map is to be specified by the user (step S1). Then, generation conditions of the electronic density map, which are specified by the user, such as the boundary value size, the voxel size, and the number of trials for each voxel size are acquired (step S2).

An electron density map obtained from the read-out X-ray scattering profile is generated in accordance with the acquired generation conditions (step S3). Details of generation of the electron density map are described later. Next, a theoretical scattering profile is calculated based on the generated electron density map (step S4). Based on the read-out measured X-ray scattering profile and the calculated theoretical scattering profile, a calculated value Rg(ind) as the particle radius of gyration for each electron density map of the objective molecule and an index $\chi^2$ representing the degree of coincidence between the measured X-ray scattering profile and the calculated X-ray scattering profile based on an electron density map are calculated (step S5), and a plot of $\chi^2$-Rg(ind) is generated (step S6).

Next, it is determined for the repetitive condition whether or not the generation of the electron density map has been tried a predetermined number of times for a particular voxel size (step S7). If it is determined that the trial has not been performed the predetermined number of times, the process returns to step S3. If it is determined that the predetermined number of trials has been performed, the process proceeds to step S8. The predetermined number of times is, for example, 50. In the above-described example, the repetitive processing is performed for a particular voxel size in the step S7, but the repetitive processing may be simply performed a predetermined number of times.

Thus, it is determined whether or not the plotted $\chi^2$-Rg(ind) generated for a particular voxel size is correlated (step S8). Details of the correlation determining process are described below. When it is determined that there is no correlation, it is determined whether or not there is any trend in the plot by multivariate analysis or the like (step S9). If it is determined that there is a trend, the process returns to step S3, and the electron density map is generated again by changing the condition. When it is determined that there is no trend, the series of processes is ended without selecting a representative of the electron density maps. When the repetitive processing of the step S7 is completed, the presence or absence of correlations is determined, and the processing with no prospect is terminated, whereby the computational resource can be efficiently used.

On the other hand, if it is determined that there is a correlation in the step S8, it is determined whether or not the condition for terminating the repetition, which the electron density maps are completed to be generated for all of the plurality of voxel sizes, is satisfied (step S10), and if it is determined that the condition is not satisfied, the voxel size is changed and the process returns to the step S3.

In the step S10, when the condition for terminating the repetition is satisfied, a regression line is obtained based on the plotting of $\chi^2$-Rg(ind) according to the aggregation of the electron-density maps, and the intercept Rg(sect) at $\chi^2$=1

of the regression line is calculated (step S11). Then, a plot of the intercept Rg(sect) with respect to the voxel size is generated (step S12), a regression line is obtained based on the plot, and a calculated value Rg(calc) of the radius of gyration of the molecule is calculated as an extrapolated section where the voxel size is zero in the regression line (step S13). On the other hand, a Guinier plot is generated for the read-out X-ray scattering profile, and an actual measurement value Rg(exp) of the radius of gyration of the molecules is calculated (step S14).

Next, it is determined whether or not the calculated value Rg(calc) is valid by determining whether or not the difference between the calculated value Rg(calc) of the radius of gyration and the actual measurement value Rg(exp) is within a certain range (step S15). At this time, when the sample forms hydration shells in the solution, it is preferable to treat a numerical value obtained by subtracting a predetermined value for the hydration shell thickness from a value obtained from the Guinier plot as the actual measurement value Rg(exp). In the case that hydration shells are formed, the predetermined value is preferably 1.5 Å to 2.0 Å. Alternatively, the thickness of the hydration shells estimated by various calculation methods for the purpose of explicitly calculating the presence of the hydration shells may be used.

In the step S15, if the calculated value Rg(calc) is not determined to be valid, the series of processes is terminated without selecting a representative one of the electron density maps. If it is determined that the calculated value Rg(calc) is valid, a representative electron density map is selected among the electron density maps having a $\chi^2$ close to 1 among the electron density maps according to the series of $\chi^2$-Rg correlations, and the selected electron density map is outputted to the outputting device 290 (step S16), and the series of processes is ended.

The representative electron density map is not necessarily single and may be plural. For example, if a plurality of electron density maps in which the plotting of $\chi^2$-Rg(ind) has a strong series of correlations and $\chi^2$ is very close to 1 are obtained, they may be selected as electron density maps corresponding to the respective canonical structure of the dynamic structure ensemble. In this case, it is desirable to select the smallest voxel size among the voxel sizes used for correlation mapping. Or, in this case, it is desirable to select the correlation defined with the smallest voxel value among correlations generated with various voxel values.

In the above example, each of the plurality of electron density maps is generated for each repetition but may be generated at once by parallel processes. Further, a certain number of parallel processes may be repeatedly performed. Which process is to be chosen can be determined based on which of the computational resources and the speed of outcome is important.

(Generation of Electron Density Map)

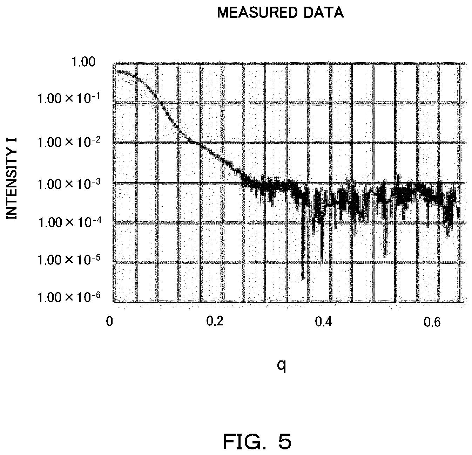

Next, the generation of the electron density map is described here in detail. Fig. 5 is a graph showing an example of an X-ray solution scattering profile. When the biomacromolecules in the solution are irradiated with X-rays, a gradual ring-shaped intensity peak of scattered X-rays is generated in the approximate region of $q \leqq 0.7$ Å$^{-1}$. By integrating it in the circumferential direction, an X-ray solution scattering profile as shown in Fig. 5 is obtained. In particular, it is possible to directly visualize the electron density that is meaningful to the actual structure by acquiring and analyzing the scattered intensity data in the q range up to 0.7 Å$^{-1}$ with high accuracy.

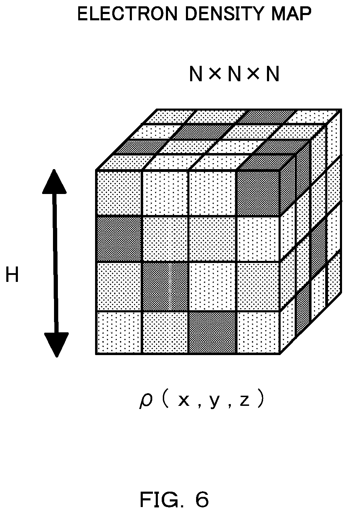

Fig. 6 is a schematic diagram showing the electron density map. In the generation of the electron density map, first, the volume of the cube box with one side H including the macromolecule in the real space is discretized into an $N \times N \times N$ grid of the voxels of the cube (N=4 in the example shown in Fig. 6). As indicated by the grayscale of each voxel in Fig. 6, the electronic density $\rho(x,y,z)$ of each voxel is randomly given a numerical value in a certain range. Then, the three-dimensional reciprocal lattice space intensities are calculated from the three-dimensional structural factors, and are divided into concentric shells as a function of the magnitude of the scattering vector q.

The three-dimensional scatter intensities are then transformed into a one-dimensional profile and compared to experimental scattering data. Three-dimensional structural factors are scaled to match the experimental data of the concentric shells of each q, and an inverse Fourier transform is performed to generate a new electron density map in real space. The density outside the map is set to zero. new structural factors are obtained by the forward Fourier transformation and this cycle is repeated until convergence. Thus, different electron density maps are generated for each trial, and index $\chi^2$ and calculated value Rg(ind) are calculated for each of the electron density maps.

(Correlation Determination Process)

By plotting the calculated $\chi^2$ and Rg(ind) for each electron density map and determining whether they are correlated with each other, it is possible to determine the validity of the electron density map with respect to the measured data. Whether or not there is a correlation in the plot can be objectively determined, for example, by using a correlation coefficient. Furthermore, when there is no correlation, by determining whether or not there is a trend in the plot, it is possible to determine whether the data is unlikely to be meaningful or can be meaningful depending on the measurement conditions.

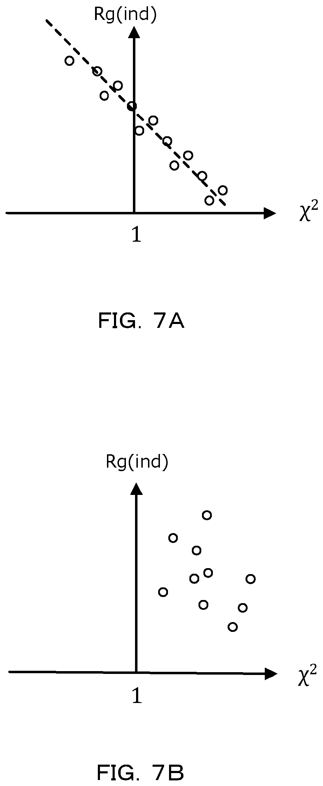

Figs. 7A and 7B are graphs showing ideal and unanalyzable distributions of $\chi^2$-Rg(ind) respectively. In the ideal distribution shown in Fig. 7A, correlations appear in the plot of $\chi^2$-Rg(ind), and it is evident that a plurality of the electron density maps are valid. On the other hand, in the distributions which cannot be analyzed shown in Fig. 7B, correlations and tendencies do not appear in the plot of $\chi^2$-Rg(ind), and it can be seen that a plurality of the electron-density maps are not valid. If the electron density maps are valid, an electron density map having a $\chi^2$ within 1 to a predetermined range can be selected as a representative electron density map.

[Example]

In practice, electron density maps were specified using samples of biomacromolecules in solutions (human serum-albumin (HSA)). Electron density maps were generated by 50 trials for each voxel size at 10 Å, 5 Å, 4 Å, and 3 Å. Then, $\chi^2$ and Rg(ind) of the generated electron density maps were calculated. Fig. 8 is a graph showing the distribution of the calculated Rg(ind) of molecular radius of gyration with respect to the index $\chi^2$ of the examples. The correlation was confirmed for each voxel size at 10 Å, 5 Å, 4 Å, and 3 Å.

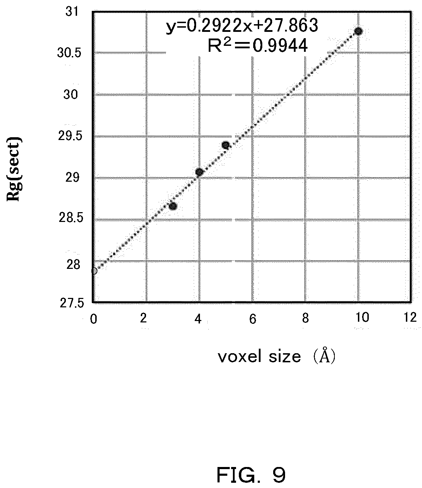

Then, the respective Rg(sect) of the voxel sizes 10 Å, 5 Å, 4 Å, and 3 Å was calculated on the basis of a straight line representing the correlation. Fig.9 is a graph showing extrapolation of Rg(sect) with respect to voxel size in the embodiment. The voxel sizes and the obtained Rg(sect) were plotted to calculate Rg(calc) for the entire plurality of the electron density maps. These results indicate that the generated electron density maps are valid.

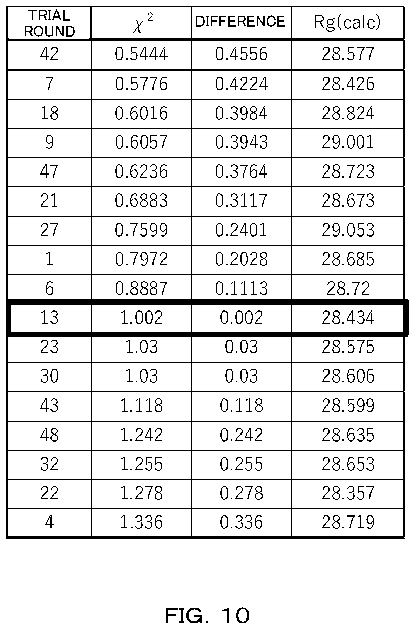

Fig. 10 is a list showing electron density maps and processing results in the example. Since more reasonable electron density maps can be obtained for a smaller voxel size, $\chi^2$ for trials of a voxel size of 3 Å are arranged in ascending order. According to the obtained list, the electron density map of the thirteenth trial with 0.002 as a difference between $\chi^2$ and 1 is most valid, so this has been selected as the representative electron density map.

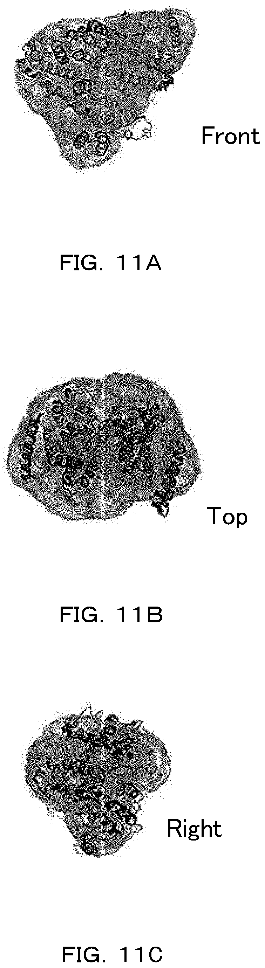

Figs. 11A to 11C are a front, plan and right side view showing the selected electron density map respectively. As described above, the electron density map in the thirteenth trial for a voxel size of 3 Å has been specified and visualized.

The visualized electron density indicates a shape like a human liver as a whole. This is a good representation of the molecular surface shape obtained from X-ray crystallography of HSA. Further, in Fig. 11A, a depression of about 5 Å and a cylindrical bulge along the depression are observed from the center of the longest side in the bottom right toward the top left. The visualized shape coincides well with the shape obtained from the X-ray structure analysis not only in terms of the overall shape but also in terms of the fine features of the molecular surface structure.

[Description of Symbols]

    10 electron density map specifying system
    100 X-ray solution scattering apparatus
    110 **X-ray** generating section
    111 **X-ray** source
    115 optical system
    117 Kratky block
    120 sample loading mechanism
    125 sample holding tube
    130 detector
    140 control unit
    200 electron density map specifying apparatus
    210 computer
    211 I/O controlling section
    215 measurement controlling section
    217 measurement data storing section
    221 electron density map generating section
    225 theoretical scattering intensity calculating section
    226 index calculating section
    231 correlation determining section
    232 trend analyzing section
    245 theoretical size calculating section
    246 actual size calculating section
    257 comprehensively judging section
    258 electron density map selecting section
    280 inputting device

290 outputting device
L control bus

**Claims**

1. An electron density map specifying apparatus (200) for specifying an electron density map of a macromolecule in a solution, said electron density map being represented by a plurality of discretized voxels, said voxels having a particular voxel size, said apparatus comprising:

   an electron density map generating section (221) for

   (i) calculating a theoretical X-ray scattering profile calculated by randomly assigning an electron density to each voxel in the real space,
   (ii) calculating scaling structure factors to fit the calculated theorectical X-ray scattering profile with an X-ray scattering profile obtained by measuring a sample,
   (iii) providing a new electron density in real space using the scaled structure factors,
   (iv) repeating the calculation of the structure factors and the electron density until convergence to generate one electron density map, and
   (v) generating the one electron density map a predetermined number of times for said particular voxel size to generate multiple electron density maps,

   an index calculating section (226) for calculating $\chi^2$ defined by formula (1) below as an index representing a degree of coincidence between the respective calculated theorectical X-ray scattering profiles and the measured X-ray scattering profile, $\chi^2$ being calculated said predetermined number of times, and
   an electron density map selecting section (258) for selecting an electron density map having a correlation between a first plot of a parameter representing a molecular size with respect to the calculated index and a regression line of the first plot and having the calculated index close to 1 as a representative electron density map from the plurality of electron density maps based on the predetermined number of calculated indexes, wherein

   $$\chi 2 = \left[ \frac{I_{exp}^{(i)}(q_j) - I_{calc}^{(i)}(q_j)}{\sigma(q_j)} \right]^2 \quad \cdots (1)$$

   where $I_{calc}(q)$ is the calculated scattering intensity, $I_{exp}(q)$ is the actual measurement value and $\sigma(q)$ is the standard deviation of $I_{exp}(q)$.

2. The electron density map specifying apparatus (200) according to claim 1, further comprising:

   a correlation determining section (231) for generating the first plot of a parameter representing a molecular size with respect to the calculated index for each of the plurality of electron density maps by using a correlation coefficient and determining the presence or absence of correlation between the first plot and the regression line thereof,
   wherein the electron density map selecting section (258) does not select the representative electron density map when there is no correlation of the first plot.

3. The electron density map specifying apparatus (200) according to claim 2, further comprising

   a trend analyzing section (232) for performing multivariate analysis on the distribution of the first plot when there is no correlation between the first plot and the regression line thereof,
   wherein the trend analyzing section (232) determines whether there is any trend in the first plot through the multivariate analysis, and
   the electron density map generating section (221) generates the electron density map again by changing the condition if it is determined that there is a trend.

4. The electron density map specifying apparatus (200) according to claim 2,

wherein the correlation determining section (231) generates an outputable first plot when there is no correlation between the first plot and the regression line thereof, and the electron density map generating section (221) generates the plurality of electron density maps under a condition based on an instruction from a user.

5. The electron density map specifying apparatus (200) according to any one of claims 1 to 4,
wherein the electron density map generating section (221) generates each of the plurality of electron density maps one by one in a repetitive process according to a setting.

6. The electron density map specifying apparatus (200) according to any one of claims 1 to 4,
wherein the electron density map generating section (221) generates each of the plurality of electron density maps at a time in parallel processes according to a setting.

7. The electron density map specifying apparatus (200) according to any one of claims 1 to 6, further comprising:

a theoretical size calculating section (245) for generating a second plot of a parameter representing a molecular size calculated based on the plurality of electron density maps with respect to each voxel size and calculating a calculated value of a parameter representing a molecular size of a macromolecule in the solution using the second plot, when each of the electron density maps is represented by a plurality of discretized voxels, and an actual size calculating section (246) for calculating a parameter representing a molecular size of a macromolecule in the solution from the measured X-ray scattering profile as an actual measurement value, wherein the electron density map selecting section (258) does not select the representative electron density map unless a difference between the calculated value and the actual measurement value is within a predetermined range.

8. A system (10) comprising an X-ray solution scattering apparatus (100) and the electron density map specifying apparatus (200) according to any one of claims 1 to 7,
wherein the electron density map specifying apparatus (200) specifies an electron density map of a macromolecule in the solution based on an X-ray scattering profile of the macromolecule in the solution measured by the X-ray solution scattering apparatus.

9. A computer-implemented method for specifying an electron density map of a macromolecule in a solution, said electron density map being represented by a plurality of discretized voxels, said voxels having a particular voxel size, the method comprising the steps of:

(i) calculating a theoretical X-ray scattering profile calculated by randomly assigning an electron density to each voxel in the real space,
(ii) calculating scaling structure factors to fit the calculated theoretical X-ray scattering profile with an X-ray scattering profile obtained by measuring a sample,
(iii) providing a new electron density in real space using the scaled structure factors,
(iv) repeating the calculation of the structure factors and the electron density until convergence to generate one electron density map,
(v) generating the one electron density map a predetermined number of times for said particular voxel size to generate multiple electron density maps,
calculating $\chi^2$ defined by formula (1) below as an index representing a degree of coincidence between the respective calculated theoretical X-ray scattering profiles and the measured X-ray scattering profile, $\chi^2$ being calculated said predetermined number of times, and
selecting an electron density map having a correlation between a first plot of a parameter representing a molecular size with respect to the calculated index and a regression line of the first plot and having the calculated index close to 1 as a representative electron density map from the plurality of electron density maps based on the predetermined number of calculated indexes, wherein

$$\chi 2 = \left[ \frac{I_{\exp}^{(i)}(q_j) - I_{\text{calc}}^{(i)}(q_j)}{\sigma(q_j)} \right]^2 \qquad \cdots (1)$$

where $I_{\text{calc}}(q)$ is the calculated scattering intensity, $I_{\exp}(q)$ is the actual measurement value and $\sigma(q)$ is the

standard deviation of $I_{exp}(q)$.

10. A computer program for specifying an electron density map of a macromolecule in a solution, the program causing a computer to carry out the method of claim 9.

**Patentansprüche**

1. Elektronendichtekarten-Spezifizierungsvorrichtung (200) zum Spezifizieren einer Elektronendichtekarte eines Makromoleküls in einer Lösung, wobei die Elektronendichtekarte durch eine Vielzahl von diskretisierten Voxeln repräsentiert ist, wobei die Voxel eine bestimmte Voxelgröße aufweisen, wobei die Vorrichtung umfasst:

   eine Elektronendichtekarten-Erzeugungssektion (221) für ein

   (i) Berechnen eines theoretischen Röntgenstreuprofils, das durch zufälliges Zuweisen einer Elektronendichte zu jedem Voxel im realen Raum berechnet wird,
   (ii) Berechnen von Skalierungsstrukturfaktoren, um das berechnete theoretische Röntgenstreuprofil mit einem Röntgenstreuprofil zu fitten, das durch Messen einer Probe erhalten wurde,
   (iii) Bereitstellen einer neuen Elektronendichte im realen Raum unter Verwendung der skalierten Strukturfaktoren,
   (iv) Wiederholen der Berechnung der Strukturfaktoren und der Elektronendichte bis zur Konvergenz, um eine Elektronendichtekarte zu erzeugen, und
   (v) Erzeugen der einen Elektronendichtekarte eine vorbestimmte Anzahl von Malen für die bestimmte Voxelgröße, um mehrere Elektronendichtekarten zu erzeugen,

   eine Indexberechnungssektion (226) zum Berechnen von $\chi^2$, das durch die nachstehende Formel (1) definiert ist, als einen Index, der einen Grad der Übereinstimmung zwischen den jeweiligen berechneten theoretischen Röntgenstreuprofilen und dem gemessenen Röntgenstreuprofil repräsentiert, wobei $\chi^2$ die vorbestimmte Anzahl von Malen berechnet wird, und
   eine Elektronendichtekarten-Auswahlsektion (258) zum Auswählen einer Elektronendichtekarte, die eine Korrelation zwischen einem ersten Diagramm eines Parameters, der eine Molekülgröße in Bezug auf den berechneten Index repräsentiert, und einer Regressionsgeraden des ersten Diagramms aufweist und deren berechneter Index nahe 1 liegt, als eine repräsentative Elektronendichtekarte aus der Vielzahl von Elektronendichtekarten basierend auf der vorbestimmten Anzahl berechneter Indizes, wobei

$$\chi 2 = \left[ \frac{I_{exp}^{(i)}(q_j) - I_{calc}^{(i)}(q_j)}{\sigma(q_j)} \right]^2 \quad \cdots (1)$$

   wobei $I_{calc}(q)$ die berechnete Streuintensität ist, $I_{exp}(q)$ der tatsächliche Messwert ist und $\sigma(q)$ die Standardabweichung von $I_{exp}(q)$ ist.

2. Elektronendichtekarten-Spezifizierungsvorrichtung (200) nach Anspruch 1, ferner umfassend:

   eine Korrelationsbestimmungssektion (231) zum Erzeugen des ersten Diagramms eines Parameters, der eine Molekülgröße in Bezug auf den berechneten Index für jede der Vielzahl von Elektronendichtekarten repräsentiert, unter Verwendung eines Korrelationskoeffizienten und zum Bestimmen des Vorhandenseins oder Nichtvorhandenseins einer Korrelation zwischen dem ersten Diagramm und dessen Regressionsgeraden,
   wobei die Elektronendichtekarten-Auswahlsektion (258) die repräsentative Elektronendichtekarte nicht auswählt, wenn keine Korrelation des ersten Diagramms vorliegt.

3. Elektronendichtekarten-Spezifizierungsvorrichtung (200) nach Anspruch 2, ferner umfassend:

   eine Trendanalyse-Sektion (232) zum Durchführen einer multivariaten Analyse der Verteilung des ersten Diagramms, wenn keine Korrelation zwischen dem ersten Diagramm und seiner Regressionsgeraden vorliegt, wobei die Trendanalyse-Sektion (232) durch die multivariate Analyse bestimmt, ob in dem ersten Diagramm ein Trend vorliegt, und

die Elektronendichtekarten-Erzeugungssektion (221) die Elektronendichtekarte erneut erzeugt, indem sie die Bedingung ändert, wenn bestimmt wird, dass ein Trend vorliegt.

4. Elektronendichtekarten-Spezifizierungsvorrichtung (200) nach Anspruch 2,
   wobei die Korrelationsbestimmungssektion (231) ein ausgabefähiges erstes Diagramm erzeugt, wenn keine Korrelation zwischen dem ersten Diagramm und seiner Regressionsgeraden besteht, und die Elektronendichtekarten-Erzeugungssektion (221) die Vielzahl von Elektronendichtekarten unter einer Bedingung basierend auf einer Anweisung eines Benutzers erzeugt.

5. Elektronendichtekarten-Spezifizierungsvorrichtung (200) nach einem der Ansprüche 1 bis 4,
   wobei die Elektronendichtekarten-Erzeugungssektion (221) jede der Vielzahl von Elektronendichtekarten nacheinander in einem sich wiederholenden Prozess gemäß einer Einstellung erzeugt.

6. Elektronendichtekarten-Spezifizierungsvorrichtung (200) nach einem der Ansprüche 1 bis 4,
   wobei die Elektronendichtekarten-Erzeugungssektion (221) jede der Vielzahl von Elektronendichtekarten gleichzeitig in parallelen Prozessen gemäß einer Einstellung erzeugt.

7. Elektronendichtekarten-Spezifizierungsvorrichtung (200) nach einem der Ansprüche 1 bis 6, ferner umfassend:

   eine Sektion (245) zum Berechnen der theoretischen Größe, um ein zweites Diagramm eines Parameters zu erzeugen, der eine Molekülgröße repräsentiert, die basierend auf der Vielzahl von Elektronendichtekarten mit Bezug auf jede Voxelgröße berechnet wird, und um einen berechneten Wert eines Parameters zu berechnen, der eine Molekülgröße eines Makromoleküls in der Lösung repräsentiert, unter Verwendung des zweiten Diagramms, wenn jede der Elektronendichtekarten durch eine Vielzahl von diskretisierten Voxeln repräsentiert wird, und
   eine Sektion (246) zum Berechnen der tatsächlichen Größe, um einen Parameter, der eine Molekülgröße eines Makromoleküls in der Lösung repräsentiert, aus dem gemessenen Röntgenstreuprofil als einen tatsächlichen Messwert zu berechnen,
   wobei die Elektronendichtekarten-Auswahlsektion (258) die repräsentative Elektronendichtekarte nur dann auswählt, wenn eine Differenz zwischen dem berechneten Wert und dem tatsächlichen Messwert innerhalb eines vorbestimmten Bereichs liegt.

8. System (10) umfassend eine Röntgenlösungsstreuvorrichtung (100) und die Elektronendichtekarten-Spezifizierungsvorrichtung (200) nach einem der Ansprüche 1 bis 7,
   wobei die Elektronendichtekarten-Spezifizierungsvorrichtung (200) eine Elektronendichtekarte eines Makromoleküls in der Lösung basierend auf einem Röntgenstreuprofil des Makromoleküls in der Lösung spezifiziert, das von der Röntgenlösungsstreuvorrichtung gemessen wurde.

9. Computerimplementiertes Verfahren zum Spezifizieren einer Elektronendichtekarte eines Makromoleküls in einer Lösung, wobei die Elektronendichtekarte durch eine Vielzahl von diskretisierten Voxeln repräsentiert wird, wobei die Voxel eine bestimmte Voxelgröße aufweisen, wobei das Verfahren die Schritte umfasst:

   (i) Berechnen eines theoretischen Röntgenstreuprofils, das durch zufälliges Zuweisen einer Elektronendichte zu jedem Voxel im realen Raum berechnet wird,
   (ii) Berechnen von Skalierungsstrukturfaktoren, um das berechnete theoretische Röntgenstreuprofil mit einem Röntgenstreuprofil zu fitten, das durch Messen einer Probe erhalten wurde,
   (iii) Bereitstellen einer neuen Elektronendichte im realen Raum unter Verwendung der skalierten Strukturfaktoren,
   (iv) Wiederholen der Berechnung der Strukturfaktoren und der Elektronendichte bis zur Konvergenz, um eine Elektronendichtekarte zu erzeugen, und
   (v) Erzeugen der einen Elektronendichtekarte eine vorbestimmte Anzahl von Malen für die bestimmte Voxelgröße, um mehrere Elektronendichtekarten zu erzeugen,
   Berechnen von $\chi^2$, das durch die nachstehende Formel (1) definiert ist, als einen Index, der einen Grad der Übereinstimmung zwischen den jeweiligen berechneten theoretischen Röntgenstreuprofilen und dem gemessenen Röntgenstreuprofil repräsentiert, wobei $\chi^2$ die vorbestimmte Anzahl von Malen berechnet wird, und

Auswählen einer Elektronendichtekarte, die eine Korrelation zwischen einem ersten Diagramm eines Parameters, der eine Molekülgröße in Bezug auf den berechneten Index repräsentiert, und einer Regressionsgeraden des ersten Diagramms aufweist und deren berechneter Index nahe 1 liegt, als eine repräsentative Elektronendichtekarte aus der Vielzahl von Elektronendichtekarten basierend auf der vorbestimmten Anzahl berechneter Indizes, wobei

$$\chi 2 = \left[ \frac{I_{\text{exp}}^{(i)}(q_j) - I_{\text{calc}}^{(i)}(q_j)}{\sigma(q_j)} \right]^2 \quad \cdots (1)$$

wobei $I_{\text{calc}}(q)$ die berechnete Streuintensität ist, $I_{\text{exp}}(q)$ der tatsächliche Messwert ist und $\sigma(q)$ die Standardabweichung von $I_{\text{exp}}(q)$ ist.

10. Computerprogramm zum Spezifizieren einer Elektronendichtekarte eines Makromoleküls in einer Lösung, wobei das Programm einen Computer veranlasst, das Verfahren nach Anspruch 9 auszuführen.


**Revendications**

1. Appareil de spécification de carte de densité électronique (200) pour la spécification d'une carte de densité électronique d'une macromolécule dans une solution, ladite carte de densité électronique étant représentée par une pluralité de voxels discrétisés, lesdits voxels ayant une taille de voxel particulière, ledit appareil comprenant :

   une section de génération de carte de densité électronique (221) pour

   (i) calculer un profil de diffraction des rayons X théorique calculé par l'attribution, de manière aléatoire, d'une densité électronique à chaque voxel dans l'espace réel,
   (ii) calculer des facteurs de structure de redimensionnement pour faire correspondre le profil de diffraction des rayons X théorique calculé avec un profil de diffraction des rayons X obtenu par la mesure d'un échantillon,
   (iii) fournir une nouvelle densité électronique dans l'espace réel à l'aide des facteurs de structure redimensionnés,
   (iv) répéter le calcul des facteurs de structure et de la densité électronique jusqu'à convergence pour générer une unique carte de densité électronique, et
   (v) générer l'unique carte de densité électronique un nombre prédéterminé de fois pour ladite taille de voxel particulière pour générer de multiples cartes de densité électronique,

   une section de calcul d'indice (226) pour calculer $\chi^2$ défini par la formule (1) ci-dessous en tant qu'indice représentant un degré de coïncidence entre les profils de diffraction des rayons X théoriques calculés et le profil de diffraction des rayons X mesuré, $\chi^2$ étant calculé ledit nombre de fois prédéterminé, et
   une section de sélection de carte de densité électronique (258) pour sélectionner une carte de densité électronique ayant une corrélation entre un premier tracé d'un paramètre représentant une taille moléculaire par rapport à l'indice calculé et une droite de régression du premier tracé et ayant l'indice calculé proche de 1 en tant que carte de densité électronique représentative parmi la pluralité de cartes de densité électronique sur la base du nombre prédéterminé d'indices calculés, dans lequel

$$\chi 2 = \left[ \frac{I_{\text{exp}}^{(i)}(q_j) - I_{\text{calc}}^{(i)}(q_j)}{\sigma(q_j)} \right]^2 \quad \cdots (1)$$

où $I_{\text{calc}}(q)$ est l'intensité de diffraction calculée, $I_{\text{exp}}(q)$ est la valeur de mesure réelle et $\sigma(q)$ est l'écart-type de $I_{\text{exp}}(q)$.

**2.** Appareil de spécification de carte de densité électronique (200) selon la revendication 1, comprenant en outre :

une section de détermination de corrélation (231) pour générer le premier tracé d'un paramètre représentant une taille moléculaire par rapport à l'indice calculé pour chacune de la pluralité de cartes de densité électronique en utilisant un coefficient de corrélation et déterminer la présence ou l'absence de corrélation entre le premier tracé et la droite de régression de celui-ci,

dans lequel la section de sélection de carte de densité électronique (258) ne sélectionne pas la carte de densité électronique représentative lorsqu'il n'existe pas de corrélation du premier tracé.

**3.** Appareil de spécification de carte de densité électronique (200) selon la revendication 2, comprenant en outre

une section d'analyse de tendance (232) pour réaliser une analyse multivariée sur la distribution du premier tracé lorsqu'il n'existe pas de corrélation entre le premier tracé et la droite de régression de celui-ci,

dans lequel la section d'analyse de tendance (232) détermine s'il existe une quelconque tendance dans le premier tracé par le biais de l'analyse multivariée, et

la section de génération de carte de densité électronique (221) génère la carte de densité électronique à nouveau en changeant la condition s'il est déterminé qu'il existe une tendance.

**4.** Appareil de spécification de carte de densité électronique (200) selon la revendication 2,

dans lequel la section de détermination de corrélation (231) génère un premier tracé apte à être délivré en sortie lorsqu'il n'existe pas de corrélation entre le premier tracé et la droite de régression de celui-ci, et la section de génération de carte de densité électronique (221) génère la pluralité de cartes de densité électronique selon une condition basée sur une instruction provenant d'un utilisateur.

**5.** Appareil de spécification de carte de densité électronique (200) selon l'une quelconque des revendications 1 à 4, dans lequel la section de génération de carte de densité électronique (221) génère chacune de la pluralité de cartes de densité électronique une par une dans un processus répétitif selon un réglage.

**6.** Appareil de spécification de carte de densité électronique (200) selon l'une quelconque des revendications 1 à 4, dans lequel la section de génération de carte de densité électronique (221) génère chacune de la pluralité de cartes de densité électronique à la fois dans des processus parallèles selon un réglage.

**7.** Appareil de spécification de carte de densité électronique (200) selon l'une quelconque des revendications 1 à 6, comprenant en outre :

une section de calcul de taille théorique (245) pour générer un second tracé d'un paramètre représentant une taille moléculaire calculée sur la base de la pluralité de cartes de densité électronique par rapport à chaque taille de voxel et calculer une valeur calculée d'un paramètre représentant une taille moléculaire d'une macromolécule dans la solution à l'aide du second tracé, lorsque chacune des cartes de densité électronique est représentée par une pluralité de voxels discrétisés, et

une section de calcul de taille réelle (246) pour calculer un paramètre représentant une taille moléculaire d'une macromolécule dans la solution à partir du profil de diffraction des rayons X mesuré en tant que valeur de mesure réelle,

dans lequel la section de sélection de carte de densité électronique (258) ne sélectionne pas la carte de densité électronique représentative sauf si une différence entre la valeur calculée et la valeur de mesure réelle est au sein d'une plage prédéterminée.

**8.** Système (10) comprenant un appareil de diffraction des rayons X en solution (100) et l'appareil de spécification de carte de densité électronique (200) selon l'une quelconque des revendications 1 à 7,

dans lequel l'appareil de spécification de carte de densité électronique (200) spécifie une carte de densité électronique d'une macromolécule dans la solution sur la base d'un profil de diffraction des rayons X de la macromolécule dans la solution mesuré par l'appareil de diffraction des rayons X en solution.

**9.** Procédé mis en œuvre par ordinateur pour la spécification d'une carte de densité électronique d'une macromolécule dans une solution, ladite carte de densité électronique étant représentée par une pluralité de voxels discrétisés, lesdits voxels ayant une taille de voxel particulière, le procédé comprenant :

(i) le calcul d'un profil de diffraction des rayons X théorique calculé par l'attribution, de manière aléatoire, d'une densité électronique à chaque voxel dans l'espace réel,

(ii) le calcul de facteurs de structure de redimensionnement pour faire correspondre le profil de diffraction des rayons X théorique calculé avec un profil de diffraction des rayons X obtenu par la mesure d'un échantillon,

(iii) la fourniture d'une nouvelle densité électronique dans l'espace réel à l'aide des facteurs de structure redimensionnés,

(iv) la répétition du calcul des facteurs de structure et de la densité électronique jusqu'à convergence pour générer une carte de densité électronique,

(v) la génération de l'unique carte de densité électronique un nombre prédéterminé de fois pour ladite taille de voxel particulière pour générer de multiples cartes de densité électronique,

le calcul de $\chi^2$ défini par la formule (1) ci-dessous en tant qu'indice représentant un degré de coïncidence entre les profils de diffraction des rayons X théoriques calculés et le profil de diffraction des rayons X mesuré, $\chi^2$ étant calculé ledit nombre de fois prédéterminé, et

la sélection d'une carte de densité électronique ayant une corrélation entre un premier tracé d'un paramètre représentant une taille moléculaire par rapport à l'indice calculé et une droite de régression du premier tracé et ayant l'indice calculé proche de 1 en tant que carte de densité électronique représentative parmi la pluralité de cartes de densité électronique sur la base du nombre prédéterminé d'indices calculés, dans lequel

$$\chi 2 = \left[ \frac{I_{exp}^{(i)}(q_j) - I_{calc}^{(i)}(q_j)}{\sigma(q_j)} \right]^2 , \qquad \cdots (1)$$

où $I_{calc}(q)$ est l'intensité de diffraction calculée, $I_{exp}(q)$ est la valeur de mesure réelle et $\sigma(q)$ est l'écart-type de $I_{exp}(q)$.

10. Programme d'ordinateur pour la spécification d'une carte de densité électronique d'une macromolécule dans une solution, le programme amenant un ordinateur à mettre en œuvre le procédé selon la revendication 9.

FIG. 1

100

111

115

117

125

130

SAMPLE

FIG. 2

FIG. 3

FIG. 4

```
                    ( START )
                        |
   S1 ──  READING OUT SCATTERING PROFILE
                        |
   S2 ──  OBTAINING CALCULATION CONDITIONS OF ELECTRON DENSITY
                        |
   S3 ──  GENERATING ELECTRON DENSITY MAPS
                        |
   S4 ──  CALCULATING THE THEORETICAL SCATTERING
          INTENSITY OF THE GENERATED ELECTRON DENSITY MAP
                        |
   S5 ──  CALCULATING χ² AND Rg(ind) FROM
          MEASURED DATA AND CALCULATED DATA
                        |
   S6 ──  PLOTTING χ²−Rg(ind)
                        |
   S7 ──  END FOR A SPECIFIC          No
          VOXEL SIZE? ──────────────────────→
                        | Yes
   S8 ──  CORRELATED?
                        |
          No ←──────────┤
                        | Yes
   S9 ──  WITH          COMPLETION             No
          TREND?  S10 ──  OF CALCULATING ──────────→
     Yes ←┤           TOTAL ELECTRON
          |           DENSITY MAP?
          | No               | Yes
          |      S11 ──  OBTAINING INTERCEPT Rg(sect)
          |                  |
          |      S12 ──  PLOTTING VOXEL SIZE−Rg(sect)
          |                  |
          |      S13 ──  CALCULATING CALCULATED Rg(calc)
          |                  |
          |      S14 ──  CALCULATING ACTUAL
          |              MEASUREMENT VALUE Rg(exp)
          |                  |
          |         No       |          S15
          ←────── VALID SIZE?
          |                  | Yes
          |      S16 ──  SELECTING AND DISPLAYING A
          |              REPRESENTATIVE ELECTRON DENSITY MAP
          |                  |
          └──────────────→ ( END )
```

MEASURED DATA

FIG. 5

ELECTRON DENSITY MAP

$N \times N \times N$

H

$\rho ( x , y , z )$

FIG. 6

FIG. 7A

FIG. 7B

FIG. 8

FIG. 9

| TRIAL ROUND | $\chi^2$ | DIFFERENCE | Rg(calc) |
|---|---|---|---|
| 42 | 0.5444 | 0.4556 | 28.577 |
| 7 | 0.5776 | 0.4224 | 28.426 |
| 18 | 0.6016 | 0.3984 | 28.824 |
| 9 | 0.6057 | 0.3943 | 29.001 |
| 47 | 0.6236 | 0.3764 | 28.723 |
| 21 | 0.6883 | 0.3117 | 28.673 |
| 27 | 0.7599 | 0.2401 | 29.053 |
| 1 | 0.7972 | 0.2028 | 28.685 |
| 6 | 0.8887 | 0.1113 | 28.72 |
| **13** | **1.002** | **0.002** | **28.434** |
| 23 | 1.03 | 0.03 | 28.575 |
| 30 | 1.03 | 0.03 | 28.606 |
| 43 | 1.118 | 0.118 | 28.599 |
| 48 | 1.242 | 0.242 | 28.635 |
| 32 | 1.255 | 0.255 | 28.653 |
| 22 | 1.278 | 0.278 | 28.357 |
| 4 | 1.336 | 0.336 | 28.719 |

FIG. 10

Front

FIG. 11A

Top

FIG. 11B

Right

FIG. 11C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **THOMAS D GRANT**. Ab initio electron density determination directly from solution scattering data. *Nature Methods*, 29 January 2018, vol. 15, 191-193

- **RONNEBAUM TREY A et al.** Higher-order oligomerization of a chimeric $\alpha\beta\gamma$ bifunctional deterpene synthase with prenyltransferase and class II cyclase activities is concentration-dependent. *Journal Of Structural Biology*, 21 January 2020, vol. 210, 1-11